**Europäisches Patentamt**

**European Patent Office**

**Office européen des brevets**

(19)

(11) Veröffentlichungsnummer : **0 372 664 B1**

(12) **EUROPÄISCHE PATENTSCHRIFT**

(45) Veröffentlichungstag der Patentschrift :
25.11.92 Patentblatt 92/48

(51) Int. Cl.⁵ : **A61L 11/00, A61L 2/06**

(21) Anmeldenummer : **89250078.6**

(22) Anmeldetag : **08.11.89**

(54) **Dampfdrucksterilisator zum Sterilisieren von Abfällen oder dergleichen.**

(30) Priorität : **02.12.88 DE 3841076**

(43) Veröffentlichungstag der Anmeldung :
**13.06.90 Patentblatt 90/24**

(45) Bekanntmachung des Hinweises auf die
Patenterteilung :
**25.11.92 Patentblatt 92/48**

(84) Benannte Vertragsstaaten :
**AT BE CH DE ES FR GB GR IT LI LU NL SE**

(56) Entgegenhaltungen :
**DE-A- 2 422 907**
**GB-A- 2 171 587**
**US-A- 4 552 720**

(73) Patentinhaber : **K-E-G**
**KRANKENHAUS-ENTSORGUNGS**
**GESELLSCHAFT m.b.H.**
**Werkring 1**
**W-1000 Berlin 20 (DE)**

(72) Erfinder : **Drauschke, Stefan**
**Triftstrasse 50**
**W-1000 Berlin 65 (DE)**
Erfinder : **Birkholz, Michaela**
**Heidestrasse 22**
**W-1000 Berlin 28 (DE)**

(74) Vertreter : **Pfenning, Meinig & Partner**
**Mozartstrasse 17**
**W-8000 München 2 (DE)**

## Beschreibung

Die Erfindung betrifft einen Dampfdrucksterilisator zum Sterilisieren von Abfällen nach dem Oberbegriff des Hauptanspruchs.

Für die Desinfektion und Sterilisation von infektiösen Abfällen bzw. für die Vernichtung von Organteilen werden im allgemeinen Sondermüllverbrennungsanlagen eingesetzt. Diese Anlagen weisen die allgemein bekannten Nachteile auf, beispielsweise tritt eine Umweltbelästigung durch die Emissionen in Form von toxischen Stoffen in Rauchgasen und gegebenenfalls unangenehmen Gerüchen bei der Verbrennung der Abfälle und durch ein Stützfeuer auf. Das Stützfeuer ist nur mit einem erheblichen Energieaufwand realisierbar, wobei bei den in der Parxis meist diskontinuierlichen Anlagen hinzukommt, daß eine Verbrennung von der Müllabfuhr nicht zuführbaren Krankenhausabfällen erst dann erfolgen kann, wenn die Anlage zuvor ausreichend aufgeheizt und damit mit Wärmeenergie beaufschlagt worden ist. Außerdem müssen in solchen Fällen die Abfälle zwischengelagert werden.

Es sind auch schon Dampfdrucksterilisatoren vorgeschlagen worden, die aber nicht zur Anwendung kamen, da üblicherweise für die Zerkleinerung ein Rührwerk in dem Sterilisator vorgesehen ist und dadurch besteht die Gefahr, daß Teile des Abfalls aus dem Inneren des Sterilisators über die Beschickungsöffnung nach außen gelangen, so daß eine höhere Infektionsgefahr besteht.

Der Erfindung liegt somit die Aufgabe zugrunde, einen Dampfdrucksterilisator zu schaffen, mit dem eine Beseitigung von Abfällen, insbesondere von infektiösen Abfällen, wie Krankenhausabfällen, möglich ist, mit dem eine wirtschaftliche Beseitigung bei hygienisch einwandfreier Beschickung realisiert werden kann.

Diese Aufgabe wird erfindungsgemäß durch die kennzeichnenden Merkmale des Hauptanspruchs, in Verbindung mit den Merkmalen des Oberbegriffs gelöst.

Dadurch, daß die Beschickungsöffnung mit einer Druckschleuse verbunden ist, die mindestens eine im Gehäuse der Druckschleuse vorgesehene Kammer führt und daß die Kammer Bestandteil einer Vorrichtung ist, die die Kammer von einer mit der mindestens einen Füllöffnung in Verbindung stehenden Füllstellung in eine mit der Beschickungsöffnung in Verbindung stehenden Beschickungsstellung bewegt, ist die Beschickungsvorrichtung Bestandteil des Sterilisators, d.h. sie ist sterilisierbar, so daß die Sterilisatoren hygienisch einwandfrei beschickt werden können, ohne daß Teile oder Wasser oder dergleichen aus dem Sterilisator herausgeschleudert werden können. Durch diese Maßnahme eröffnet sich die Möglichkeit der Verwendung von Großraum-Sterilisatoren zur Beseitigung von infektiösen Materialien, wie Krankenhausabfälle, die wesentliche wirtschaftliche Vorteile bringt.

Durch die in den Unteransprüchen angegebenen Maßnahmen sind vorteilhafte Weiterbildungen und Verbesserungen möglich. Durch Vorsehen einer Drehvorrichtung als Bestandteil der Druckschleuse, in der die eine oder mehrere Kammern angeordnet sind, kann die gesamte Beschickungsvorrichtung relativ einfach und platzsparend aufgebaut werden.

Ein Ausführungsbeispiel der Erfindung ist in der Zeichnung dargestellt und wird in der nachfolgenden Beschreibung näher erläutert.

Es zeigen:

Fig. 1 eine Seitenansicht auf einen Teil des Sterilisators mit Beschickungsvorrichtung und

Fig. 2 eine Aufsicht gemäß der Schnittlinie A-A nach Fig. 1.

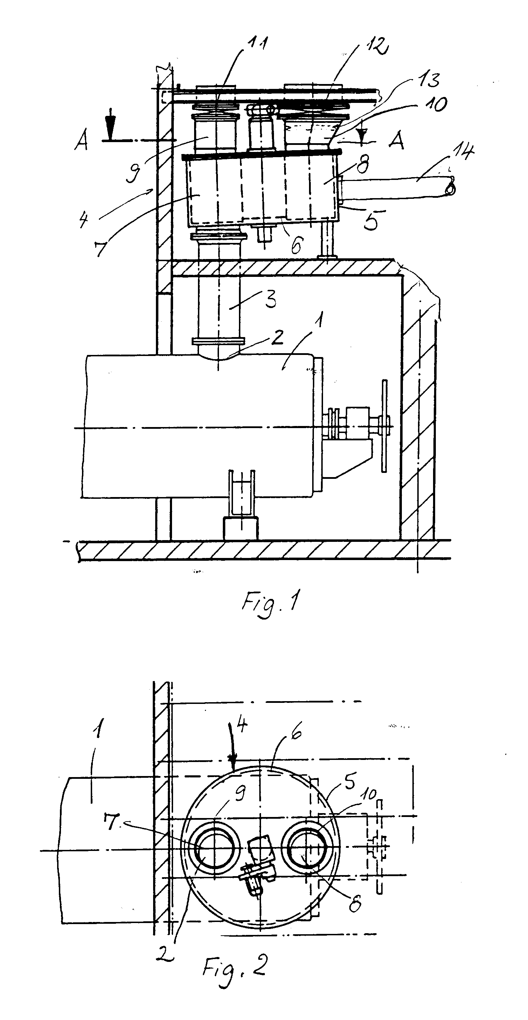

In Fig. 1 ist mit 1 ein Sterilisator oder Autoklav bezeichnet, der üblicherweise mit Rührwerken ausgerüstet ist, um den zu sterilisierenden Abfall oder dergleichen zu homogenisieren, aufzuheizen und umzuwälzen. Zur Sterilisation seines Inhalts wird der Sterilisator 1 mit Prozeßdampf beaufschlagt. Der Sterilisator 1 ist mit einer Beschickungsöffnung 2 versehen, die mit einem rohr- oder trichterförmigen Beschickungskanal 3 verbunden ist. Der Beschickungskanal 3 ist Bestandteil einer als Druckschleuse ausgebildeten Beschickungsvorrichtung 4, die ein Gehäuse 5 in Zylinderform aufweist, in dem eine um den Mittelpunkt bewegbare Drehvorrichtung 6 bzw. ein Rondell gelagert ist. In der Drehvorrichtung sind eine Mehrzahl von Kammern 7, 8 über ihren Umfang ringförmig angeordnet, wobei nur zwei zu sehen sind, die zur Aufnahme von in Behältern aufgenommenen infektiösen und/oder nassen organischen Materialien dient. Die Kammern 7, 8 sind nach unten hin beispielsweise mit einem Schieber abgeschlossen, der bei Drehung in die Stellung, in der eine Kammer 7, 8 über dem Beschickungskanal 3 liegt, geöffnet wird, so daß die jeweilige Kammer in den Sterilisator 1 entleert wird. Die Kammern 7, 8 können auch nach unten hin offen sein, wobei dann allerdings die Behälter an dem Gehäuseboden schleifen. Es ist auch denkbar, daß lediglich ein Verschluß über dem Beschickungskanal vorgesehen ist. Die Beschickungsvorrichtung 4 weist zwei diametral entgegengesetzte Füllöffnungen 9, 10 auf, die in eine jeweils darunterliegende Kammer 7, 8 münden, wobei im Ausführungsbeispiel eine Füllöffnung 9 in Verlängerung zum Beschickungsschacht 3 und der jeweils darüber angeordneten Kammer 7 liegt. Die Füllöffnungen 9, 10 sind mittels Verschlüssen druckdicht abgeschlossen, wobei die Verschlüsse als Schieber ausgebildet sein können.

Die Drehvorrichtung 6 wird über einen nicht dargestellten elektrischen Antrieb und eine entsprechende Übersetzungsanordnung 11 angetrieben, wobei ein drehzahlverstellbares Getriebe vorgesehen ist, über das die Drehzahl des Rondells bzw. der Drehvorrichtung 6 einstellbar ist.

Das Gehäuse und die Drehvorrichtung 6 der Beschickungsvorrichtung 4 sind zum Beschickungskanal 3 hin etwas geneigt, um Kondensat abfließen zu lassen.

In den Füllöffnungen 9,10, gegebenenfalls auch in den Kammern 7,8 und/oder dem Beschickungskanal 3 können Reinigungsdüsen oder dergleichen Reinigungsvorrichtung angeordnet sein. Beispielsweise kann in der Füllöffnung 10 ein Düsenkranz 13 integriert sein, durch den Reinigungsflüssigkeit zugeführt wird, die durch die Beschickungsvorrichtung strömt und diese reinigt und dann in den Sterilisator 1 abfließt. Gleichzeitig kann der Düsenkranz 13 dazu dienen, einen auf die Füllöffnung 10 gestülpten Wechselbehälter auszuspritzen und zu reinigen, nachdem dieser entleert worden ist. Wenn ein Wechselbehälter benutzt wird, werden die infektiösen und-/oder nassen organischen Abfälle in Säcke abgefüllt und in den Wechselbehälter transportiert.

Vorzugsweise ist das Gehäuse an ein Luft- oder Gasabsaugrohr 14 angeschlossen, über das die Luft oder das Gas abgesaugt wird, damit beim Befüllen diese nicht nach außen dringen. Die über das Absaugrohr 14 abgesaugte Luft wird zur Reinigung über ein Keimfilter geführt.

Zum Befüllen der Kammern 7,8 und zum Beschicken des Sterilisators 1 werden die druckdichten Verschlüsse 11, 12 bzw. der druckdichte Verschluß 12 geöffnet und die Kammer 8 wird mit infektiösen und/oder nassen organischen Materialien gefüllt bzw. der Behälter wird direkt über die Füllöffnung 11, die darunterliegende Kammer 7 und den Beschickungskanal 3 dem Sterilisator 1 zugeführt. Die Drehvorrichtung 6 dreht sich weiter, so daß eine andere Kammer unterhalb der Füllöffnung 12 bzw. 11 liegt, so daß die Befüllung wiederholt werden kann. Wenn eine gefüllte Kammer 7,8 in die Beschickungsstellung über den Beschickungskanal gedreht wird, öffnet sich bei Vorhandensein von Schiebern mit der Drehung der Schieber oder dergleichen am Boden der Kammer, so daß sie vollends nach unten hin geöffnet ist, wenn sie mit dem Beschickungskanal 3 fluchtet. Die Behälter fallen senkrecht in den Sterilisator und werden dort durch das Rührwerk erfaßt. Dabei können keine Teile des infektiösen oder nassen organischen Materials oder Wasser nach außen herausgeschleudert werden, da die Beschickungsvorrichtung 4 diese zurückhält. Die möglicherweise im Beschickungskanal 3 oder anderswo anhaftenden Teile können durch die Reinigungsdüsen entfernt werden. Für die Sterilisation sowohl des Materials im Sterilisator 1 als auch der Beschickungsvorrichtung 4 selbst werden die Verschlüsse 11,12 druckdicht verschlossen und die gesamte Anordnung wird mit dem Prozeßdampf desinfiziert bzw. sterilisiert, wobei anschließend die Beschickung erneut vorgenommen werden kann. Dabei ist die als Druckschleuse ausgebildete Beschickungsvorrichtung 4 wie der Sterilisator 1 selbst für einen Betriebsdruck von 4 bar ausgelegt.

Die Drehung der Drehvorrichtung 6, deren Drehzahl gesteuert werden kann, kann kontinuierlich oder auch schrittweise in Intervallen erfolgen.

In dem Ausführungsbeispiel ist die Beschickungsvorrichtung 4 als Drehvorrichtung ausgebildet. Es ist aber auch denkbar, daß eine Verschiebevorrichtung vorgesehen ist, wobei dann die Kammern in Längsrichtung nebeneinander angeordnet sind und die Verschiebbewegung translatorisch erfolgt.

**Patentansprüche**

1. Dampfdrucksterilisator zum Sterilisieren von Abfällen oder dergleichen, der vorzugsweise mit einem Rührwerk ausgerüstet ist und eine Beschickungsöffnung aufweist,
   **dadurch gekennzeichnet,**
   daß die Beschickungsöffnung (2) mit einer Druckschleuse (4) verbunden ist, die mindestens eine druckdicht zu verschließende, in mindestens eine im Gehäuse (5) der Druckschleuse (4) vorgesehene Kammer (7,8) führende Füllöffnung (12) aufweist, und daß die Kammer (7,8) Bestandteil einer Vorrichtung (6) ist, die die Kammer (7,8) von einer mit der mindestens einen Füllöffnung (12) in Verbindung stehenden Füllstellung in eine mit der Beschickungsöffnung (2) in Verbindung stehenden Beschickungsstellung bewegt.

2. Dampfdrucksterilisator nach Anspruch 1, dadurch gekennzeichnet, daß die Vorrichtung als Drehvorrichtung (6) oder Rondell ausgebildet ist.

3. Dampfdrucksterilisator nach Anspruch 1 oder 2, dadurch gekennzeichnet, daß eine Mehrzahl von Kammern (7,8) vorgesehen ist.

4. Dampfdrucksterilisator nach einem der Ansprüche 1 bis 3, dadurch gekennzeichnet, daß die Kammer (7,8) mit einer Verschlußvorrichtung versehen ist, die in der Füllstellung geschlossen ist und bei Bewegung in die Beschickungsstellung öffnet.

5. Dampfdrucksterilisator nach einem der Ansprüche 1 bis 4, dadurch gekennzeichnet, daß zwischen Kammer (7) und Beschickungsöffnung (2) ein Beschickungskanal (3) vorgesehen ist.

6. Dampfdrucksterilisator nach Anspruch 5, dadurch gekennzeichnet, daß in der mindestens einen Füllöffnung und/oder mindestens einen Kammer und/oder dem Beschickungskanal (3) eine Reinigungsvorrichtung (13), wie Reinigungsdüsen, vorgesehen ist.

7. Dampfdrucksterilisator nach einem der Ansprüche 1 bis 6, dadurch gekennzeichnet, daß eine weitere Füllöffnung (11) vorgesehen ist, die in der Verlängerung zur Beschickungsöffnung (2) und zur in der Beschickungsstellung liegenden Kammer (7) angeordnet ist.

8. Dampfdrucksterilisator nach einem der Ansprüche 1 bis 7, dadurch gekennzeichnet, daß ein elektrischer Antrieb zum Bewegen der die mindestens eine Kammer aufweisenden Vorrichtung in die unterschiedlichen Stellungen vorgesehen ist.

9. Dampfdrucksterilisator nach Anspruch 8, dadurch gekennzeichnet, daß der elektrische Antrieb drehzahlverstellbar ist.

10. Dampfdrucksterilisator nach einem der Ansprüche 1 bis 9, dadurch gekennzeichnet, daß die Druckschleuse (4) mit einer Absaugvorrichtung (14) verbunden ist, die Luft bzw. Gas absaugt.

## Claims

1. Autoclave for the sterilization of waste or the like, which is preferably equipped with an agitator and which comprises a feed opening, **characterised in that** the feed opening (2) is connected to a pressure gate (4) which comprises at least one charge opening (12), which can be closed to be pressure-tight and which connects to at least one pressure gate (4) in the housing (5), and that the chamber (7, 8) is part of a device (6) which moves the chamber (7, 8) from a charge position which connects to at least one charge opening (12) into a feed position which connects to the feed opening (2).

2. Autoclave according to claim 1, **characterised in that** the device is arranged as a rotary device (6) or a rondel.

3. Autoclave according to claim 1 or 2, **characterised in that** a plurality of chambers (7, 8) is provided.

4. Autoclave according to one of claims 1 to 3, **characterised in that** the chamber (7, 8) is provided with a locking device which is shut in the charge position and which opens when moved into a feed position.

5. Autoclave according to one of claims 1 to 4, **characterised in that** a feed channel (3) is provided between the chamber (7) and the feed opening (2).

6. Autoclave according to claim 5, **characterised in that** a cleaning device (13), such as cleaning nozzles, is provided in the at least one charge opening and/or at least one chamber and/or the feed channel (3).

7. Autoclave according to one of claims 1 to 6, **characterised in that** an additional charge opening (11) is provided, which is arranged in an extension to the feed opening (2) and to the chamber (7) located in the feed position.

8. Autoclave according to one of claims 1 to 7, **characterised in that** an electrical drive is provided for moving the device which comprises the at least one chamber into the different positions.

9. Autoclave according to claim 8, **characterised in that** the electrical drive is speed-adjustable.

10. Autoclave according to one of claims 1 to 9, **characterised in that** the pressure gate (4) is connected to a syphoning device (14) which syphons off air or gas respectively.

## Revendications

1. Autoclave de stérilisation de déchets ou produits similaires, de préférence équipé d'un dispositif mélangeur et présentant une ouverture de chargement, caractérisé en ce que l'ouverture de chargement (2) est reliée à un sas de chargement sous pression (4) qui présente au moins une ouverture de remplissage (12) munie d'une fermeture étanche à la pression, aboutissant dans au moins une chambre (7, 8) prévue dans le carter dudit sas (4) de chargement et en ce que la chambre (7, 8) fait partie intégrante d'un dispositif (6) qui la déplace d'une position de remplissage située en rapport avec ladite ouverture de remplissage (12), vers une position de chargement située en rapport avec ladite ouverture de chargement (2).

2. Autoclave de stérilisation selon la revendication 1, caractérisé en ce que ledit dispositif est réalisé sous la forme d'un appareil tournant (6) ou carrousel.

3. Autoclave de stérilisation selon l'une quelconque des revendications 1 ou 2, caractérisé en ce qu'on a prévu une pluralité de chambres (7, 8).

4. Autoclave de stérilisation selon l'une quelconque des revendications 1 à 3, caractérisé en ce que la chambre (7, 8) est pourvue d'un dispositif de fermeture qui est fermé dans la position de remplissage (12) et qui s'ouvre lors du déplacement dans la position de chargement.

5. Autoclave de stérilisation selon l'une quelconque des revendications 1 à 4, caractérisé en ce qu'un canal de chargement (3) est prévu entre la chambre (7) et l'ouverture de chargement (2).

6. Autoclave de stérilisation selon la revendication 5, caractérisé en ce qu'un dispositif de nettoyage (13) tel que des buses de nettoyage est prévu dans au moins l'une des ouvertures de remplissage et/ou au moins dans l'une des chambres et/ou dans ledit canal de chargement (3).

7. Autoclave de stérilisation selon l'une quelconque des revendications 1 à 6, caractérisé en ce qu'il est prévu une autre ouverture de remplissage (11) disposée dans le prolongement de l'ouverture de chargement (2) et dans celui de la chambre (7) situé en position de chargement.

8. Autoclave se stérilisation selon l'une quelconque des revendications 1 à 7, caractérisé en ce qu'un dispositif d'entraînement électrique est prévu pour déplacer le dispositif muni d'au moins une chambre dans les différentes positions.

9. Autoclave de stérilisation selon la revendication 8, caractérisé en ce que le dispositif d'entraînement électrique est réglable en vitesse de rotation.

10. Autoclave de stérilisation selon l'une quelconque des revendications 1 à 9, caractérisé en ce que le sas de chargement (4) sous pression est reliée à un dispositif d'aspiration (14) qui aspire de l'air ou du gaz.

Fig. 1

Fig. 2